# EUROPEAN PATENT SPECIFICATION

(11) **EP 3 116 555 B1**
(45) Date of publication and mention of the grant of the patent: **25.08.2021**
(21) Application number: 15713393.5
(22) Date of filing: 05.03.2015
(51) Int. Cl.: A61L 27/12, A61L 27/52, A61L 27/54, A61L 31/02, A61L 31/14, A61L 31/16

(54) **ACTIVE AGENT-PARTICLE COMBINATION SUPPORTING BONE REGENERATION**
WIRKSTOFF-PARTIKEL-KOMBINATION ZUR UNTERSTÜTZUNG DER KNOCHENREGENERATION
COMBINAISON AGENT ACTIF-PARTICULE FAVORISANT LA RÉGÉNÉRATION OSSEUSE

(30) Priority: 14.03.2014 WO PCT/IB2014/059786; 23.10.2014 WO PCT/IB2014/065560
(43) Date of publication of application: 18.01.2017
(73) Proprietor: Ecole Polytechnique Fédérale de Lausanne (EPFL), 1015 Lausanne (CH)
(72) Inventor: KETTENBERGER, Ulrike, CH-1006 Lausanne (CH); PIOLETTI, Dominique, CH-1164 Buchillon (CH)
(74) Representative: Grosfillier, Philippe
(86) International application number: PCT/EP2015/054576
(87) International publication number: WO 2015/135822

(56) References cited:
- US-A1- 2005 282 783
- US-A1- 2006 257 492
- None

## Description

### Technical Field

The present invention relates to compositions useful in the biomedical field, in particular as biomaterials for tissue regeneration, especially for bone tissues.

### Background Art

The high regenerative capacity of bone means that the majority of fractures will heal well without the need for major intervention. Despite this, large bone defects, as observed after bone tumor resections and severe non-union fractures, lack the template for an orchestrated regeneration and require surgical intervention. Currently, the gold standard treatment is the use of a procedure called autografting, which involves the harvest of' donor' bone from a non-load-bearing site in the patient (typically an easily accessible site like the iliac crest) and transplantation into the defect site.

Transplanting autologous bone (i.e. bone from the patient) has the best clinical outcome as it integrates reliably with host bone and lacks the immune- and disease-related complications of allogeneic bone (i.e. bone from a human cadaver) or xenogeneic bone (i.e. bone from an animal source). Nevertheless, its use is severely hampered by its short supply and the considerable donor site morbidity associated with the harvest.

Furthermore, many conditions like osteoporosis, bone tumors, avascular necrosis or infections could need a more invasive approach, such as the implantation of osteosynthesis devices and endoprostheses. Impaired bone quality is a serious problem in such cases, since it could bring to implant failure, mainly due to issues related to the bone stock surrounding the implant like low bone density, excessive bone resorption or an insufficient vascularization. In these cases, it is crucial to preserve and regenerate the bone stock as fast and efficient as possible for a successful outcome for the patient.

The search for new bone regeneration strategies is therefore a key priority fueled by the debilitating pain associated with bone damage, and the increasing medical and socioeconomic challenge of our aging population. Regenerative medicine has turned to new less invasive measures to heal severe bone defects with a reduced risk of infection and rejection. While materials intended for implantation were in the past designed to be 'bioinert', materials scientists have now shifted toward the design of 'bioactive' materials that integrate with biological molecules or cells and regenerate tissues. Bone and tissue formation has been encouraged by adding biological and therapeutic agents to an implantable substrate. However, many of these agents have to be properly incorporated into or onto the substrate in order to be clinically efficacious. A major problem for the clinical use of these agents is an appropriate delivery system. An ideal delivery system is one that is capable of maintaining an agent in situ for sufficient time for it to interact with target cells and for the agent to be released at an effective, but safe concentration during tissue repair/healing. In the case of bone, materials should preferably be both osteoinductive (capable of promoting the differentiation of progenitor cells down an osteoblastic lineage), osteoconductive (support bone growth and encourage the ingrowth of surrounding bone), and capable of osseointegration (integrate into surrounding bone). Many bone substitute materials intended to replace the need for autologous or allogeneic bone have been evaluated. In general, they consist of either bioactive ceramics, bioactive glasses, biological or synthetic polymers, and composites of these. The ideal basic premise, if following the tissue engineering paradigm, is that the materials will be resorbed and replaced over time by, and in tune with, the body's own newly regenerated biological tissue.

Synthetic injectable scaffolds must be able to provide structural stability and mechanical support to a defect site. The scaffolds must degrade at a sufficient rate since they are only temporary structures. The degradation rate of scaffolds is vital because it must maintain its integrity long enough for new tissue to replace damaged tissue, while simultaneously providing mechanical strength to help absorb some, but not all, of the stress. Some of the advantages of gel polymers are ability to take the shape of any size defect and ability to integrate numerous agents easily within a gel.

### Bioactive inorganic materials

A wide range of bioactive inorganic materials similar in composition to the mineral phase of bone are of clinical interest, e.g. tricalcium phosphate, hydroxyapatite (HA), bioactive glasses, and their combinations. Bioactive glasses (Ca- and possibly P-containing silica glasses), for example, when immersed in biological fluid, can rapidly produce a bioactive hydroxycarbonated apatite layer that can bond to biological tissue. Furthermore, they can be tailored to deliver ions such as Si at levels capable of activating complex gene transduction pathways, leading to enhanced cell differentiation and osteogenesis. The resorption rate of bioactive glasses and bioceramics can be tailored with crystalline HA persisting for years following implantation, while other calcium phosphates have a greater capacity to be resorbed but less strength for sustaining load. The brittle nature of bioactive inorganic materials means that their fracture toughness cannot match that of bone and on their own are not good for load-bearing applications.

### Polymers

Biological polymers, such as collagen and hyaluronic acid, are interesting candidates for tissue engineering and provide innate biological informational guidance to cells that favours cell attachment and promotes chemotactic responses. Synthetic polymers such as polyfumarates, polylactic acid (PLA), polyglycolic acid (PGA), copolymers of PLA and PGA (PLGA), and polycaprolactone offer a versatile alternative. They can be processed to generate a range of three-dimensional scaffolds with different porosities and surface characteristics. Hydrogels (e.g. polyethylene glycol, alginate-based) are also popular as they can often be delivered in a minimally invasive manner and gelled in situ (e.g. photocrosslinked or ionically) to provide a three-dimensional cellular microenvironment with high water content. Their viscoelastic material properties seem particularly suitable for cartilage regeneration, although many applications in bone have also been explored. Hydrogels have the advantage that chemical biofunctionalization and material encapsulation and delivery are relatively straightforward.

### Hyaluronan hydrogels

Hyaluronan is a natural glycosaminoglycan found in all connective tissues and the synovial fluid of joints. Its natural composition and abundance make it the subject of intense research for clinical application. Hyaluronan' s biodegradable properties, natural make up, stability to hydrolysis, cell adhesion, cell migration, cell proliferation, and cellular differentiation characteristics are important for its potential use as a tissue engineering construct. Studies with hyaluronan have shown that the material is immunologically inert and 100% biodegradable both in vitro and in vivo. Similarly, in vivo, hyaluronan has the ability to aid in the construction of a extracellular matrix, which produces a suitable and structurally protected microenvironment for progenitor cell differentiation. The ability to replicate a natural environment for progenitor cells is important, particularly due to the ease with which differentiated cells lose their functional attributes. Hydrogels are allowing seeded cells to spread throughout the material.

Hyaluronan-based injectable hydrogels have been used as a depot for small molecule drugs, such as bisphosphonate, and DNA plasmids. In addition, hyaluronan gels were used as a carrier for growth factors, such as BMPs delivery to promote bone augmentation.

### Composite materials

Inorganic-organic composites aiming at 'mimic' the composite nature of real bone combine the toughness of a polymer phase with the compressive strength of an inorganic one to generate bioactive materials with improved mechanical properties and degradation profiles. For such composites, the alkalinity of the inorganic filler neutralizes acidic autocatalytic degradation of polymers such as PLA. There is a growing recognition that a nano-sized inorganic component is likely to be more bioactive than a micro-sized one.

The biomaterials used for manufacturing dental implants include metals, ceramics, carbons, polymers, and combinations of these. Polymers are softer and more flexible than the other classes of biomaterials. They also present with low mechanical strength, which makes them prone to mechanical fractures during function under high loading forces. Polymeric materials were reported to have very little application in implant dentistry and were only used to fabricate shock-absorbing components placed between the implant and the suprastructure.

Titanium, including alloy Ti-6AI-4V (Ti-6 aluminum-4 vanadium), is the first modern material used for dental implants, and it is still one of the most used in contemporary dental implants. Commercially pure Ti is a light metal with excellent biocompatibility, relatively high stiffness and high resistance to corrosion. However, when exposed to air, a surface oxide is formed and this layer of oxide determines the biological response. This oxide layer is a dynamic interface that acts as platform for the apposition of bone matrix.

Other metals have been used for osseointegration, including zirconium, gold and Ti-aluminum-vanadium alloys. These alloys may strengthen the implant but have been shown to have relatively poor bone-to-implant contact.

Bioceramics such as hydroxyapatite are also used because of their low strength, excellent biocompatibility, and capacity to integrate with hard tissue and living bone. Besides their brittle nature, hydroxyapatite, tricalcium phosphate, and aluminum oxide ceramics are currently used as plasma-sprayed coatings onto a metallic core. This results in union of the implant with the host tissue.

Many attempts were undertaken in the past, with various success rates, to restore bone defects and allow a functional tissue regeneration of bone lesions, taking advantage from the unique features of biomaterials, and composite biomaterials in particular.

WO 2013/165333 discloses a microporous material for bone repair and bone void filling. The material comprises bioresorbale polymer granules and a biocompatible water-miscible solvent, wherein the solvent at least partially dissolves and/or softens the polymer granules to form a mouldable mass that can be used to fill a bone defect, but which hardens when the implant material is exposed to water. In the presence of water or an aqueous environment, such as being placed in the body, the solvent is removed and the implant material hardens into a mass with interconnected macroporosity.

WO 2012/117260 discloses a synthetic bone substitute, comprising a mixture of osteoconductive particles of first and second average particle sizes, suspended in a water-soluble reverse-phase hydrogel carrier, wherein the hydrogel is preferably a poloxamer.

WO 2003/043576 discloses a flowable composition for introduction to a bone or cartilage defect comprising an osteogenic agent or chondrogenic agent, where the osteogenic agent or chondrogenic is an inhibitor of bone resorption or a bone anabolic agent, and a biocompatible carrier that is more fluid at ambient temperature than at an elevated temperature.

WO 2007/134465 discloses a solid precursor for the preparation of a pasty bone replacement material by admixture of a liquid, whereby said precursor remains stable prior to use and in particular retains its molecular integrity to a high degree. Said precursor comprises a hydrogel or a substance which can be swelled into a hydrogel and calcium-containing ceramic particles.

US 2006/0257492 discloses methods and bioactive compositions for localized delivery of therapeutic agents using a suspension of porous, crystalline biomimetic particles with at least one therapeutic agent dispersed within and throughout the apatite particles. The particles of the suspension are sufficiently small that the particles and the therapeutic agent can be delivered to the subject by injection.

US 8697107 discloses a flowable biomedical implant for application to a bone defect to promote bone growth. The flowable biomedical implant comprises a carrier matrix including a biodegradable polysaccharide and ceramic particles. An impermeable membrane can be integrally formed at the surface of the carrier matrix by contacting the biodegradable polysaccharide including the ceramic particles with 0.1% by weight to about 20% by weight of a crosslinking agent. The resulting membrane forms a seal inhibiting soft tissue cells from growing into the bone defect that can reduce or prevent bone growth in the bone defect.

Shi et al. reported a study on fabrication and in vitro use of poly (lactic-co-glycolic acid) (PLGA)-hybridizing-hydroxyapatite (HA) microspheres loaded with bisphosphonate drug aledronate. The in situ aledronate delivery system inhibits the growth of macrophages while enhancing the proliferation and commitment of osteoblasts.

Nejadnik et al. showed that bisphosphonate-functionalized hyaluronic acid containing calcium phosphate particles is stable in vivo and attracts bone ingrowth. In the study, no release of the bisphosphonate molecules used to functionalize the hyluronic acid carrier is reported.

Boanini et al. reported the results of an in vitro study carried out on cultures of osteoclast and osteoblast grown on hydroxyapatite-aledronate nanocrystals. The results demonstrate that alendronate is able to influence bone cells even in composite nanocrystals.

Despite all these advancements in the technology for improvement of bone quality in the presence of structural or functional alterations, there is still a need for a valid alternative biomaterial which could permit a robust and durable restoration of bone defects and/or preserve and regenerate the bone stock, while avoiding the drawbacks associated with the known therapeutic or surgical approaches.

### Summary of invention

Accordingly, it is an object of the present invention to provide a bioactive composition having osteoconductive and osteointegrative characteristics, which can rapidly mineralize in vivo and which could serve as in situ scaffold encouraging new bone formation.

A further object of the invention is to provide a bioactive composition which preserves the existing bone stock from resorption and at the same time inhibits the degradation of the in situ forming mineralized structure.

A further object of the invention is to provide a bioactive composition which can seal bone voids and defects (e.g. resorption "crater" around dental implant following a local infection) and therefore prevent an ingrowth of fibrous tissue.

A further object of the invention is to provide a bioactive composition which improves the primary stability of implants directly after surgery.

All these aims are accomplished through the compositions herein disclosed and defined in the claims. The scope of the invention is defined by the claims. Any references in the description to methods of treatments refer to the compositions of the present invention for use in a method for treatment of the human or animal body by therapy. The invention is based, at least in part, on a surprising synergistic effect provided by the combination of an active agent loaded on calcium phosphate solid particles in promoting bone tissue repair, healing and regeneration. In particular, the invention discloses a bioactive composition comprising a carrier and solid particles loaded with an active agent.

In one aspect, the bioactive composition of the invention is characterized in that the solid particles are selected from the group consisting of crystals, granules, round particles, spherical particles or any combination thereof.

In a preferred aspect, the bioactive composition of the invention is characterized in that the solid particles are calcium phosphate solid particles.

The bioactive composition of the invention is characterized in that the carrier comprises a polymeric matrix. In a preferred aspect, the bioactive composition of the invention is characterized in that the active agent is an anti-resorptive agent.

The bioactive composition of the invention is characterized in that the solid particles have a mean particle 150 nm and 500 nm.

In a particular aspect, the bioactive composition of the invention is characterized in that it is in hydrogel form.

In a particular aspect, the bioactive composition of the invention is provided in an injectable formulation.

A further object of the present invention relies in a pharmaceutical formulation comprising the bioactive composition of the invention.

### Brief description of drawings

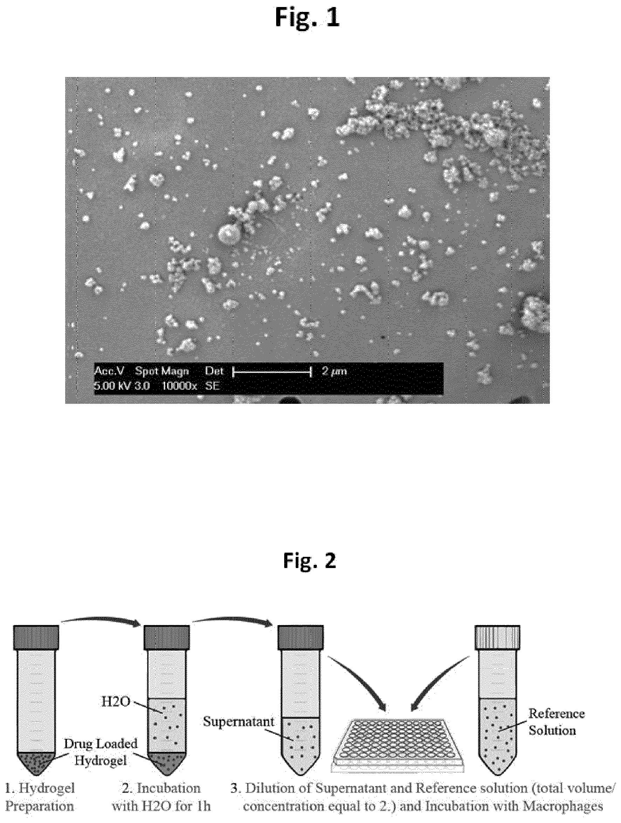

**Fig. 1** depicts hydroxyapatite nanoparticles.
**Fig. 2** depicts the preparation of the indirect release study using a RAW 264.7 macrophages cell assay. The drug-loaded hydrogel with and without HA particles was incubated in H₂O for 1 hour to investigate if a burst release of the drug occurs. The macrophages were later on exposed to the supernatants of the hydrogels that were highly diluted with cell culture medium.

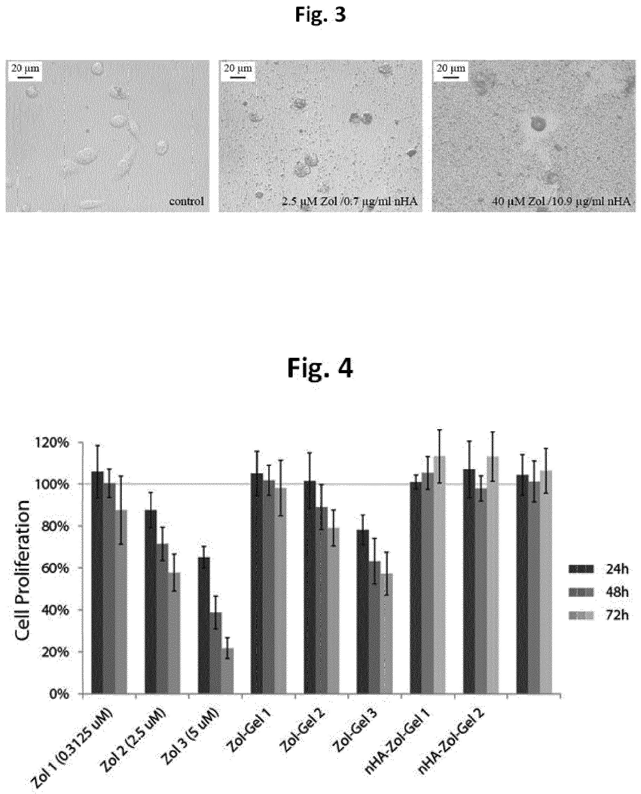

**Fig. 3** depicts microscopy images of RAW 264.7 murine macrophages after 48 h of cultivation with various concentrations of Zoledronate and HA nanoparticles. Left box: control; middle box: 2.5 µM Zoledronate - 0.7 µg/ml hydroxyapatite nanoparticles; right box: 40 µM Zoledronate - 10.9 µg/ml hydroxyapatite nanoparticles.
**Fig. 4** depicts results for the RAW 264.7 cell assay investigating the Zoledronate release from hydrogels during 1 hour of incubation in water. RAW 264.7 cells were cultivated for 24-72 h.

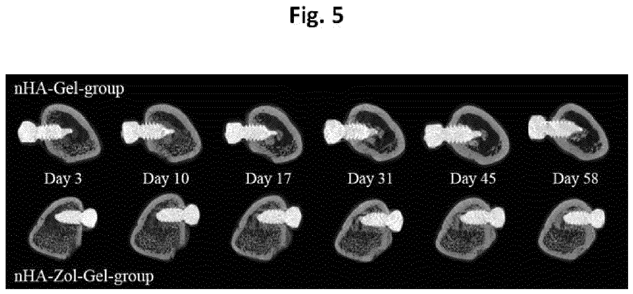

**Fig. 5** depicts MicroCT scans taken at 6 time points after surgery from the nHA-Gel-group (top), and the nHA-Zol-Gel-group (bottom). The scans show a rapid mineralization of the tissue around the screws in both groups that is resorbed and remodeled quickly in the nHA-Gel-group and persists in the nHA-Zol-Gel-group.

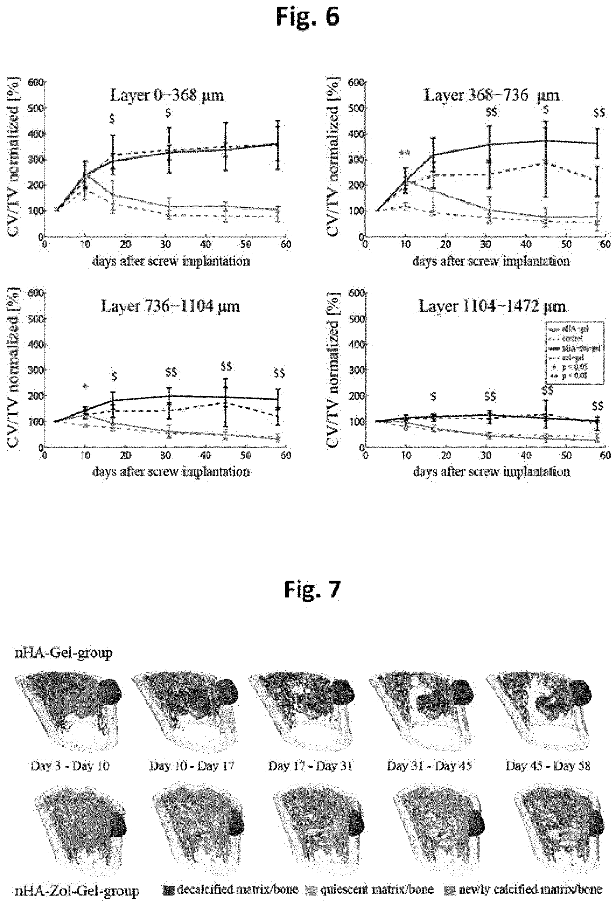

**Fig. 6** depicts mineralized volume/ tissue volume measured in 4 layers around the screw. The grey asterisks indicate the significant differences between nHA-Gel-group and control-group, the black ones between nHA-Zol-Gel-group and Zol-Gel-group and the dollar signs indicate the significant differences between nHA-Zol-Gel-group and nHA-Gel-group. The data from the Zol-Gel-group and the control group were acquired during an earlier study and are shown only form comparison reasons. Grey solid line: nHA-gel; grey dotted line: control; black solid line: nHA-Zol-gel; black dotted line: Zol-gel; *: p<0.05; **: p<0.01.
**Fig. 7** depicts volume rendered comparisons between always 2 consecutive microCT scans taken from two representative samples from the two experimental groups, one from the nHA-Gel-group (top) and one from the Zol-Gel-group (bottom).

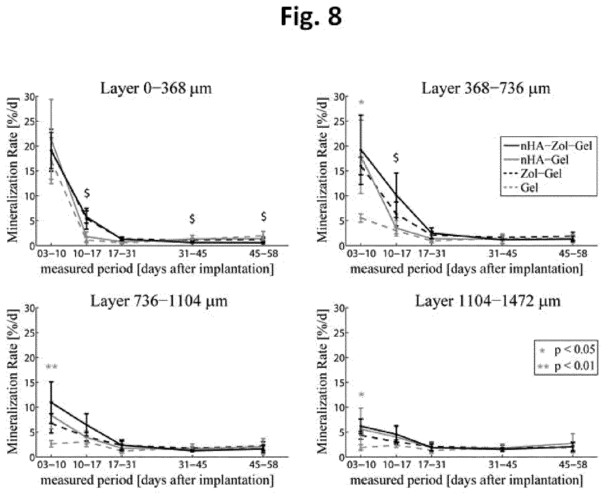

**Fig. 8** depicts mineralization rate including bone formation and biomaterial mineralization measured in 4 layers around the screw. Dollar signs indicate the significant differences between nHA-Zol-Gel-group and nHA-Gel-group. Asterisks indicate the significant differences between nHA-Gel-group and Gel-group, no significant differences were found for the nHA-Zol-Gel-group and the Zol-Gel-group. The data from the Zol-Gel-group and the control group were acquired during an earlier study and are shown only form comparison reasons. Grey solid line: nHA-gel ; grey dotted line : control; black solid line : nHA-Zol-gel; black dotted line: Zol-gel; *: p<0.05; **: p<0.01.

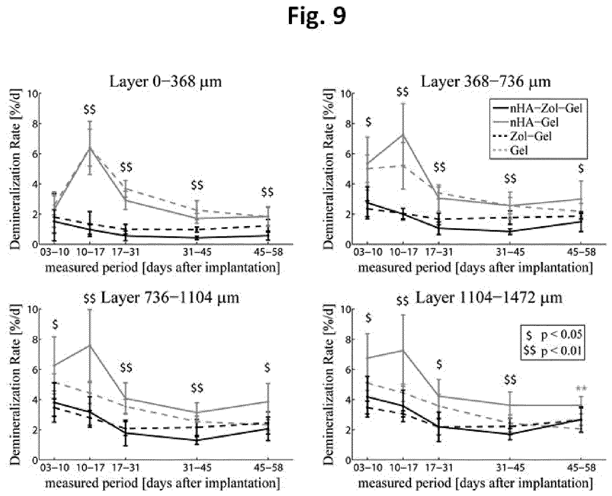

**Fig. 9** depicts demineralization rate including bone resorption and biomaterial degradation measured in 4 layers around the screw. Dollar signs indicate the significant differences between nHA-Zol-Gel-group and nHA-Gel-group. Asterisks indicate significant differences between nHA-Gel-group and Gel-group, no significant differences were found for the nHA-Zol-Gel-group and the Zol-Gel-group. The data from the Zol-Gel-group and the control group were acquired during an earlier study and are shown only form comparison reasons. Grey solid line: nHA-gel ; grey dotted line : control; black solid line : nHA-Zol-gel; black dotted line: Zol-gel; *: p<0.05; **: p<0.01.

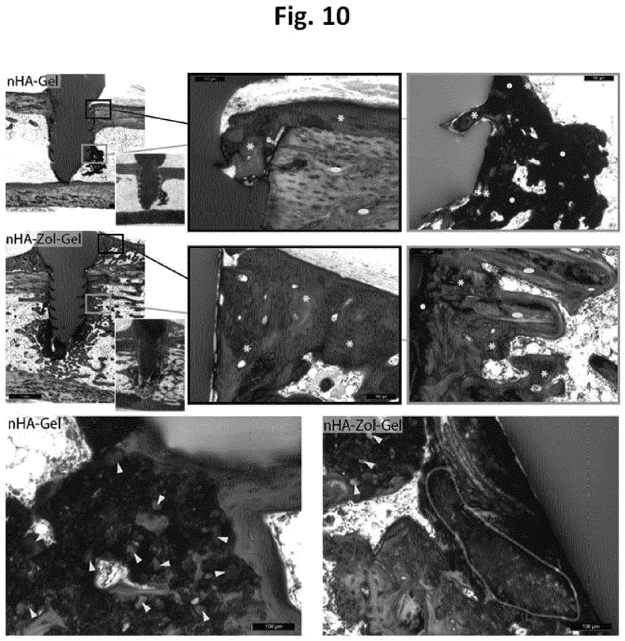

**Fig. 10** depicts histological bone sections of treated animals. Top and middle row: Toluidine blue stained ground sections from the nHA-Gel-group (top) and the nHA-Zol-Gel-group (middle). The asterisks indicate immature, non-remodeled bone areas, the ellipses mark remaining old bone. Mineralizations are highlighted with polygons. The little-boxed images are X-rays of the histology slides and show the mineralization of each region. Bottom row: details from the above shown samples stained with Giemsa for a better distinction between bone and mineralizations. In the left image from the nHA-Gel-group, small granule-shaped mineralizations are marked with arrows. In the right one from the nHA-Zol-Gel group, an extended mineralized region is marked with a line; smaller ones are also highlighted with arrows.

### Detailed description of the invention

The present disclosure may be more readily understood by reference to the following detailed description presented in connection with the accompanying drawing figures, which form a part of this disclosure. It is to be understood that this disclosure is not limited to the specific conditions or parameters described and/or shown herein, and that the terminology used herein is for the purpose of describing particular embodiments by way of example only and is not intended to be limiting of the claimed disclosure.

As used herein and in the appended claims, the singular forms "a," "and," and "the" include plural referents unless the context clearly dictates otherwise. Thus, for example, reference to "a particle" includes a plurality of such particles and reference to "an agent" includes reference to one or more agents, and so forth.

Also, the use of "or" means "and/or" unless stated otherwise. Similarly, "comprise," "comprises," "comprising" "include," "includes," and "including" are interchangeable and not intended to be limiting. It is to be further understood that where descriptions of various embodiments use the term "comprising", those skilled in the art would understand that in some specific instances, an embodiment can be alternatively described using language "consisting essentially of" or "consisting of."

In the frame of the present disclosure, a "bioactive composition" is any composition comprising an active agent. The expression "active agent", as well as "bioactive compound" or "therapeutic agent", refers to any agent that is biologically active, i.e. having an effect upon a living organism, tissue, or cell. The expression is used herein to refer to a compound or entity that alters, inhibits, activates, or otherwise affects biological or chemical events.

One skilled in the art will appreciate that a variety of therapeutic agents can be used depending upon the condition to be treated. Exemplary therapeutic agents include, but are not limited to, a growth factor, a protein, a peptide, an enzyme, an antibody, an antigen, a nucleic acid sequence (e.g., DNA or RNA), a hormone, an anti-inflammatory agent, an anti-viral agent, an anti-bacterial agent, a cytokine, an oncogene, a tumor suppressor, a transmembrane receptor, a protein receptor, a serum protein, an adhesion molecule, a neurotransmitter, a morphogenetic protein, a differentiation factor, an analgesic, a matrix protein, a cell, and combinations thereof. In other embodiments, the therapeutic agent can be an osteoinductive agent, an osteoconductive agent, an anti-resorptive agent, organic molecules, polysaccharides or any combination thereof.

An anti-resorptive agent according to the present disclosure is any agent that inhibits bone resorption, such as agents that interfere with inflammation or agents that inhibit osteoclast activity (anti-osteoclastic agent). Most important agents belonging to the group of anti-resorptive agents are bisphosphonates. Bisphosphonates are structural analogs of pyrophosphates. They have a pharmacologic activity specific for bone, due to the strong chemical affinity of bisphosphonates for hydroxyapatite. In a preferred embodiment, the active agent is a bisphosphonate such as Alendronate, Risedronate, Etidronate, Ibandronate, Pamidronate, Zoledronate or combinations thereof.

The active agent of the composition of the present invention is loaded on solid particles. It can be chemically linked to the surface of the particles, embedded in them or placed in direct proximity to them. The term "particle" is used herein to refer to any three-dimensional body, regardless of its dimensions and shape. The term includes granules, grains, powders, crystals, spherical or generally round particles and the like. In a preferred aspect of the invention, the particles are round particles. The particles have a mean particle size between 150nm and 500nm, even more preferably between 175nm and 250nm. In a most preferred embodiment, particles have a mean particle size of 200nm. Particle size refers to the length of the longest dimension of the particles. Combination of particles having a different size and shape can be envisaged.

The solid particles are calcium phosphate particles. A non-exhaustive list of calcium phosphate particles includes apatite particles, hydroxyapatite particles, calcium-deficient hydroxyapatite particles, carbonated apatite particles, flouride-apatite particles, chloride-apatite particles, tetracalcium-phosphate particles, oxyapatite particles, amorphous calcium phosphate particles, monocalcium phosphate monohydrate particles, monocalcium phosphate anhydrous particles, dicalcium phosphate dehydrate particles, dicalcium anhydrous particles, octacalcium phosphate particles, tricalcium phosphate particles or any combination thereof.

The bioactive composition of the present invention comprises a carrier. As used herein, a "carrier" is any substance which function as a dispersing means for the active agent-loaded solid particles and which allows a suitable delivery means for these latter. The carrier comprises a polymeric matrix, i.e. organised or amorphous network composed of monomeric elements.

The carrier of the composition of the present invention is a hydrogel.

As used herein, the term "gel" refers to a nonfluid colloidal network or polymer network that is expanded throughout its whole volume by a fluid. A gel is a solid three-dimensional network that spans the volume of a liquid medium and ensnares it through surface tension effects. The internal network structure may result from physical bonds (physical gels) or chemical bonds (chemical gels).

As used herein, the term "hydrogel" refers to a gel in which the swelling agent is water. A hydrogel is a macromolecular polymer gel constructed of a network of crosslinked polymer chains. It is synthesized from hydrophilic monomers, sometimes found as a colloidal gel in which water is the dispersion medium. Hydrogels are highly absorbent (they can contain over 90% water) natural or synthetic polymeric networks. As a result of their characteristics, hydrogels develop typical firm yet elastic mechanical properties.

Several physical properties of the hydrogels are dependent upon concentration. Increase in hydrogel concentration may change the hydrogel pore radius, morphology, or its permeability to different molecular weight proteins. One skilled in the art will appreciate that the volume or dimensions (length, width, and thickness) of the hydrogel of the bioactive composition can be selected based on the region or environment into which the hydrogel is to be implanted. The mechanical properties of the material can be tailored according to the application site by changing the hydrogel (molecular chain length, crosslinking, water content) and/or its composition (ratio particles/hydrogel, size of the particles, shape of the particles).

The hydrogel comprises or consists of hyaluronic acid. The hyaluronic acid consists of glucuronic acid and acetylglucosamine which create the disaccharide hyaluronic acid. The hyaluronic acid has a fibrous, non-branched molecular structure and therefore results in highly viscous liquid solutions. Hyaluronic acid's properties can be significantly improved with crosslinking, producing a hydrogel. This added feature allows to form a desired shape, as well as to deliver therapeutic molecules, into a host. Hyaluronic acid can be crosslinked by attaching thiols, methacrylates, hexadecylamides or and tyramine. Hyaluronan can also be crosslinked directly with formaldehyde or with divinylsulfone. The hydrogel may also comprise hyaluronic acid in the form of sodium hyaluronate.

The bioactive composition can be delivered or applied to a specific region in a subject. Thus, in a particular aspect, the invention pertains to the localized delivery or application of the bioactive composition of the invention to a target body region in a subject. The small size and porous nature of calcium phosphate particles in the bioactive composition lead to strong adsorption of therapeutic agents at high affinity, as well as the subsequent slow release of the therapeutic agent. The bioactive composition is characterized by its ability to interact with and adsorb therapeutic agents, which make it an ideal delivery vehicle for said agents to a target region in a subject.

The bioactive composition releases the active agent at the target region of the subject in a controlled manner and eliminates the adverse effects arising from systemic administration of the therapeutic agent. In particular, the use of the bioactive composition of the invention can avoid "burst release" of the therapeutic agent and reduces the cost incurred by systematic administration of expensive therapeutic agents. The methods and compositions of the invention also provide a minimally invasive technique for the treatment of difficult clinical situations, such as healing of large bone defects.

The local delivery of the bioactive composition, along with the controlled release of the therapeutic agent to a localized region in a subject, can be used to promote the natural bone remodelling process in that region. For example, the therapeutic agent can be an anti-resorptive agent such as bisphosphonates coupled to the solid particles, which can be incorporated into the bioactive composition. Bisphosphonates administered orally have the problem of poor gastro-intestinal absorption and need for high doses. Furthermore, some data indicated that esophagitis is a potentially serious side effect that occurs in a small percentage of patients. These problems thus present a limitation on the use of bisphosphonates according to this mode of therapy and to a certain extent, where local problem is addressed, there is a clear need to accomplish the drug delivery locally. The localized delivery of the active agent through the compositions of the invention has the advantages to avoid the adverse effects of systematic administration by locally providing the desired concentration of the agent at the target site. The efficacy of the therapeutic agent is further enhanced by controlling the absorption and desorption of the therapeutic agent such that it is released at a controlled rate. This allows the agents to be delivered at the target site at the optimum dosage for bone healing, regeneration or repair.

The composition of the invention can further comprise at least one additional therapeutic agent, e.g. bone reinforcing structures, bone growth promoting compounds or one or more additional therapeutic agents for treating a condition in which use of the bioactive composition is beneficial to amelioration of said condition. For example, when the bioactive composition is used to support bone regeneration, repair and healing in case of an osteoporosis-related fracture (with or without the need of implantation of an endoprostheses), the pharmaceutical composition can further include one or more additional therapeutic agents for treating osteoporosis, or antibiotics can be added to the composition when e.g. this is used for the treatment of bone craters around dental implants in the presence of peri-implantitis.

The bioactive composition described herein is useful in a variety of diseases, disorders, and defects where new bone formation and/or inhibition of bone resorption are an essential part of the therapy. The bioactive composition is useful for long bone defects such as in the femur, tibia, fibula, and humerus and also for vertebral body defects. The composition is also particularly useful in periodontal diseases where the alveolar bone requires additional new bone growth to support dental implants. Therefore, the bioactive composition may be utilized for a variety of orthopedic, maxillo-facial and dental surgical procedures such as the repair of simple and compound fractures, non-unions requiring external or internal fixation, joint reconstructions and total joint replacements, repairs of the vertebral column including spinal fusion and internal fixation, tumor surgery, repair of spinal injuries, and spinal deformities, such as scoliosis, intramedullary fixation of fractures, mentoplasty, temporomandibular joint replacement, alveolar ridge augmentation and reconstruction, inlay bone grafts, implant placement and revision and the like.

Typically preferred compositions are in the form of injectable hydrogels.

The composition can moreover be filled in cavities present in non-biodegradable bone implants or surgical tools or applied to the surface of those devices. An application of the composition according to the present invention through implants presenting channels or grooves such as cannulated screws can be imagined as well. Furthermore, the composition can be embedded in existing biodegradable implants like resorbable screws or plates, or added to existing formulations like biodegradable bone cements, rinsing solutions or implant coatings.

In a preferred embodiment, the bioactive composition is formulated into an injectable form. If required, the bioactive composition can be mixed with an amount of water or physiologically compatible buffer sufficient to produce the desired consistency for injection. Most often this will be as thick as possible while still being able to pass through a 16-18 gauge syringe. Other gauged syringes may also be used such as a 12-14 gauge syringe, as well as larger structures such as catheters, cannulas or large dosing tips when applying the composition to e.g. superficial bone defects like dental implant craters. For some formulations requiring injection directly into solid tissue (into e.g. cancellous bone of an osteoporosis patient), thinner consistencies (e.g., 1.5 ml H₂O/g bioactive composition) may be used. In a preferred embodiment, the mode of administration is through in situ injection (i.e., injection of the composition directly in the bone area to be treated).

The invention also provides pharmaceutical compositions comprising the bioactive composition of the invention. In another embodiment, pharmaceutical compositions of the present invention may, optionally and additionally, comprise a pharmaceutically acceptable carrier, excipient and/or diluent. As used herein, "pharmaceutically acceptable carrier" includes any and all solvents, dispersion media, coatings, antibacterial and antifungal agents, isotonic and absorption delaying agents and the like, that are physiologically compatible. Examples of suitable pharmaceutical carriers are well known in the art and include sodium chloride solutions, phosphate buffered sodium chloride solutions, water, emulsions, such as oil/water emulsions, various types of wetting agents, sterile solutions, organic solvents and so forth. The carrier suitably contains minor amounts of additives such as substances that enhance isotonicity and chemical stability. Such materials are non-toxic to recipients at the dosages and concentrations employed, and include buffers such as e.g. phosphate, citrate, succinate, acetic acid, and other organic acids or their salts; antioxidants such as ascorbic acid; low molecular weight (less than about ten residues) (poly)peptides, e.g., polyarginine or tripeptides; proteins such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

### EXAMPLES

Bisphosphonates (hereinafter, "BPs") have usually been absorbed in the past on hydroxyapatite (hereinafter, "HA") bulk implants or coatings using the high affinity of the drug to mineral. Binding BPs to bulk hydroxyapatite is associated with a slow release of the drug as a low pH is required for a spontaneous release of the drug from a mineral surface. However, a fast availability of the BP seems to be important for a positive effect on early bone formation. The inventors had therefore the intuition to combine the BPs with small enough HA-particles that can be distributed easily in bone and be uptaken by osteoclasts. Through experiments *in vitro* in cell assays and *in vivo* in an osteoporotic rat model, the inventors unexpectedly found synergistic effects on peri-implant bone remodeling by using a composition comprising osteoconductive hydroxyapatite nano-sized particles loaded with the bisphosphonate Zoledronate and a hyaluronic acid hydrogel. The present invention was based, at least in part, on earlier research performed in the inventors' lab that showed that Zoledronate locally delivered from a hyaluronic acid hydrogel increases the early bone formation rate up to 100% while efficiently inhibiting peri-implant bone resorption. Astonishingly, the main finding of the present invention was however not directly related to the anti-resorptive bisphosphonate Zoledronate. The in vivo study in a rat model of postmenopausal osteoporosis showed an unexpected rapid mineralization of the HA-particles loaded hydrogel already between day 3 and 10 after implantation, with and without the presence of Zoledronate. The drug showed its strong effect later during the study and efficiently inhibited the resorption of present and newly formed bone as well as the degradation of the biomaterial.

### Materials and Methods

Zoldedronate used for this study was bought from Enzo Life Sciences (Art.-Nr. ALX-430-153-0000, Enzo Life Sciences, Farmingdale, USA) and hydroxyapatite powder (nHA) with a particle size of less than 200 nm was bought from Sigma (art-nr. 677418, Sigma-Aldrich, St. Louis, MO, USA). Those particles have a round structure (Fig. 1). The nHA was heat sterilized before use. The cell culture medium (CCM) for the *in vitro* studies was prepared by mixing Dulbecco's Modified Eagle Medium (DMEM, Gibco) with 10% fetal bovine serum (Sigma, St. Louis, MO, USA) and 1% penicillin/streptoycin.

### In Vitro Cell Assay

Murine macrophages (RAW 264.7, passage 13) were used for the present study as those cells have been shown to react *in vitro* to bisphosphonate exposure in a similar way as osteoclasts and to tolerate well the presence of HA particles. An aqueous Zoledronate solution with a concentration of 2mg/ml was prepared and sterile filtered. The drug solution was diluted with CCM to a final concentration of 80 µM of Zoledronate. A further dilution with CCM gave Zoledronate supplemented medium (Zol-CCM) with concentrations of 80 µM, 40 µM, 20 µM, 10 µM, 5 µM, 2.5 µM and 1.25 µM. The dilution procedure was repeated with an aqueous dispersion containing 2mg/ml Zoledronate and 20 mg/ml nHA (nHA-Zol-CCM). The macrophages were seeded with a density of 2500 cell per well in 96-well plates (Microtest™ 96, Becton Dickinson Labware, NJ, USA) and incubated with 100 µl CCM at standard incubation conditions (37°C, 5% CO₂). After 24 h of incubation, 100 µl of CCM, Zol-CCM or nHA-Zol-CCM were added to the cells, the final Zoledronate concentrations in the CCM were therefore 40 µM, 20 µM, 10 µM, 5 µM, 2.5 µM, 1.25 µM and 0.625 µ M. A cell proliferation assay (CellTiter 96® AQueous One Solution, Promega, WI, USA) was performed after 24, 48 and 72 hours of cell exposure to the modified CCM. The absorbance was measured with a plate reader (Wallac 1420 Victor 2, Perkin Elmer, Waltham, MA, USA). Background plates with only the modified media were measured at all time points to avoid a bias due to the nHA particles in the dispersions.

### Hydrogel Preparation

A commercially available cross-linked hyaluronic acid (Termira AuxiGel™, Stockholm, Sweden) was used as the carrier matrix for the BP. The preparation of the hydrogel was done under sterile conditions in a laminar flow chapel. An aqueous solution containing 2 mg/ml Zoledronate was prepared and sterile filtered. The nHA was added to the solution with a nHA/Zoledronate ratio of 100:1. The exact drug dose was ensured by mixing precisely weighed and measured amounts of Zoledronate, nHA, and distilled water. A crosslinking of the hydrogel was achieved by mixing a hyaluronan derivative (component A) with a PVA cross-linker (component B). Five parts of the Zoledronate/nHA solution were mixed with 3 parts component A and 2 parts component B for the nHA-Zol-Gel-group. The Zoledronate-nHA dispersion was replaced by bi-distilled water with nHA for the nHA-Gel-group. The resulting gels were allowed to settle for 1 h before filling the capillary pistons of a positive displacement pipette (Microman®, Gilson, Middleton, USA) with 5 µl gel each. The capillaries were pulled off the pipette with the gel inside and left 1 h for settling. Then they were sterile packed in plastic tubes and frozen at -20°C.

### Drug Release Study

The release of Zoledronate from hydrogel containing nHA particles and pure hydrogel was assessed indirectly with a cell assay. Zoledronate loaded hydrogel was prepared with and without nHA following the above-described technique. The gels were incubated for 1h in bi-distilled water at room temperature (Fig. 2). The ratio of water and gel was 3:1 to reach a total concentration of 250 µg per 1 ml liquid/gel volume. For comparison, an aqueous Zoledronate solution with a concentration of 250 µl/l was also prepared (Fig. 2). The Zoledronate solution was sterile filtered and diluted in CCM to reach final concentrations of 10 µM, 5 µM, and 0.625 µM. The supernatant from the hydrogel incubation was diluted similarly. RAW 264.7 cells were prepared as previously described. After 24 h of incubation in CCM, 100 µl of the prepared media were added, the final concentrations of Zoledronate were therefore 5 µM, 2.5 µM and 0.3125 µM. A cell proliferation assay was performed after 24, 48 and 72 hours of incubation with Zoledronate.

### In Vivo Study

The in vivo study in a rat femoral model of postmenopausal osteoporosis was performed following a well- established protocol. All animal procedures were approved by the local animal care and use committee (license no. 2508.1, EXPANIM, SCAV, Epalinges, Switzerland).

### Surgical procedures

Eight virgin female Wistar rats (Janvier Labs, Saint-Berthevin, France) were ovariectomized bilaterally with a dorsal approach at an age of 17 weeks and a weight of 295±18 g to induce an estrogen deficiency related bone loss. The rats were housed 4 per cage under 12 h light to 12 h dark cycles at 22°C room temperature with 55% humidity. They were fed with a standard rodent diet (KLIBA NAFAG 3436, Provimi Kliba AG, Switzerland) and tab water at libitum. After ovariectomy (OVX) of the rats, food intake was limited to 50 g per kg body weight per day. Sterilized hay, paper tunnels and wooden sticks were offered as cage environment enrichment. The animals were fed in groups, therefore an equal nutrition of all animals was ensured by a close monitoring and regular weighing. At an age of 22 weeks and a weight of 350±20 g, miniature screws were implanted bilaterally in the femoral condyles of the rats. The screws with a thread length of 3 mm and a diameter of 1.4 mm were custom made from radiopaque polyetheretherketone (PEEK) (RISystem, Davos, Switzerland) and coated with a 100 nm titanium layer to mimic the interface with a standard orthopaedic screw. Before screw insertion, the pre-drilled unicortical screw holes (diameter 1.2mm, depth 3.5mm) were filled with 5 µl of the prepared hydrogel containing only nHA (nHA-Gel-group) or nHA and 5 µg of Zoledronate (nHA-Zol-Gel-group) with a positive displacement pipette. Both legs of each animal were treated with the same hydrogel to avoid an unwanted drug effect on the contralateral side.

### In vivo microCT imaging and image processing

In vivo microCT scans (Skyscan 1076, Bruker microCT, Kontich, Belgium) of the right femur only were performed one day before OVX and one day before screw implantation in order to confirm the bone loss caused by the estrogen deficiency. Both femurs were then scanned at day 3, 10, 17, 31, 45, and 58 after screw implantation for the dynamic histomorphometry.

The parameters for data acquisition and reconstruction were chosen according to a previously tested protocol. The animals were kept under Isoflurane anesthesia during the scanning time to avoid motion artifacts. All rats were sacrificed at the time of the last microCT scan with Pentobarbital (Esconarkon, Strauli Pharma SA, Uznach) while still being anaesthetized. The image taken for the evaluation of the bone loss and for dynamic histomorphometry were processed and analyzed using the softwares Amira® (FEI Vizualisation Sciences Group, Burlington, USA) and CTan (Bruker microCT, Kontich, Belgium). Dynamic histomorphometry is an image processing technique based on a registration and comparison of consecutive microCT scans. Mineralized tissue volume that is present only on the first microCT scan is considered as being resorbed/degraded, whilst that present only on the second scan is considered as newly mineralized tissue/formed bone. For an analysis of the spatio-temporal effect, the static and dynamic bone parameters were analyzed in 4 layers of 368 µm each around the screw. This partition has been shown before to give a good idea of the peri-implant bone healing and the normal bone remodeling.

### Histology

The rat femurs were dissected just after sacrifice and fixed, dehydrated and embedded in PMMA. After polymerization, the sections were cut, glued to PMMA slides and polished before etching their surface with 0.7 % formic acid (Applichem, Gatersleben, Germany) and staining them with Giemsa (VWR, Dietikon, Switzerland) or Toluidine Blue (VWR, Dietikon, Switzerland). Microscopy images for evaluation were taken using an upright microscope (DM 5500, Leica Microsystems, Wetzlar, Germany). The ground sections were finally X-rayed (Skyscan 1076, Bruker microCT, Kontich, Belgium) for a mapping of the tissue density.

### Statistics

Statistical testing was done with Matlab (Mathworks, Natick MA, USA). Values lying outside an interval of 1.5 times the quartile range were identified as outliers and excluded. The results of the OVX scans were analyzed with a paired t-test after performing a Lilliefors tests for testing normal distribution. In case the normal distribution could not be confirmed, a Wilcoxon singed rank test was used. The static and dynamic mineralized tissue parameters were tested for significance with a non-parametric Kruskal Wallis ANOVA followed by a Tukey's HSD (Honestly Significant Difference) test as normal distribution and equal variance could not be confirmed in all groups.

### Example 1

### In vitro effect on macrophages

The cell proliferation assay that was performed with murine RAW 264.7 macrophages showed a dose-depended proliferation decrease for cells that were exposed to Zoledronate concentrations from 0.16 to 40 µM (Fig. 3). This inhibitory effect was significantly enhanced by the presence of hydroxyapatite nanoparticles that were added to the solution with a nHA/Zoledronate ratio of 10:1. Due to the high affinity of Zoledronate to hydroxyapatite, it must be assumed that most of the drug was absorbed to the particles and therefore not present in solution. Light microscopy images showed that the macrophages incorporated the Zoledronate-loaded particles surrounding them. This finding suggests that the cells have uptaken the drug together with the nHA particles. Particles have been shown to be incorporated by macrophages via pinocytosis only for sizes smaller 500 nm, larger particles are phagocytized. The particles used for this study have a size of less than 200 nm but form agglomerations, so that both mechanisms have to be considered. In order to rule out a negative effect of the nHA powder itself on the macrophages, we also cultured them only with particles. The results show that the cells tolerated well the presence of the particles. This finding is in accordance with literature as other studies have shown the HA particles with different sizes and shapes do not considerably impact the viability of RAW 264.7 macrophages up to a concentration of 500 µg/ml.

### Example 2

### Drug Release Study

The cell proliferation test with murine macrophages only allows a rough estimation of the very early passive release from the hyaluronic acid hydrogel. The drug concentration in the medium of the two release groups (Zol-Gel, nHA-Zol-Gel) was estimated by comparing their cell proliferation with the reference group (Zol) where a known amount of Zoledronate had been put. When comparing the Zol-Gel-group with the nHA-Zol-Gel group, a clear difference can be seen between the cell proliferation (Fig. 4). By comparing the curves, it can be estimated that the final concentration of Zoledronate in the CCM of the Zol-Gel-group must have been around 75% (50%-100%) of the maximum possible concentrations that would be seen if the drug would not at all be retained by the hydrogel (Zol-group). This result confirms the assumption that the highly porous hydrogel delivers the small Zoledronate molecules via a burst release. In contrary, the diluted nHA-Zol-Gel-supernatant did not show a significant difference to control at any time and any concentration. This result suggests that no significant amount of drug has been released during the first hour of incubation in water, and that there is therefore a change of the release properties of Zoledronate due to the presence of nHA particles. As the supernatant was filtered before adding it to the CCM, it can only be concluded that no Zoledronate was in solution after 1 hour of incubation in H₂O.

### Example 3

### In vivo study

All rats accepted both surgeries well and returned to normal activity right after surgery. The limited food intake allowed a controlled weight gain of the animals; their final mean weight was 384±24 g. Analysis of the bone parameters based on the microCT scans performed before and after ovariectomy confirmed a successful ovariectomy by showing a bone loss in all animals similar to the results found before in this model.

### MicroCT based dynamic histomorphometry

A total of 87 microCT scans were analyzed for the dynamic histomorphometry as 3 had to be excluded due to motion artefacts. One leg of one animal from the nHA-Zol-Gel-group showed an excessive bone formation and was therefore also excluded. Five to eight samples were left in each group after removal of the outliers. The rapidity of the appearance and the structure of the forming mineralized tissue that became visible on the microCT scans suggested that it cannot only be formed bone (Fig. 5). Histology confirmed later that the hydrogel-nHA composite calcifies *in vivo.* As it is impossible to distinguish on the microCT scans between bone and hydroxyapatite, inventors rather analyzed mineralization rate (CR) and demineralization rate (DR) instead of bone formation and bone resorption rate and mineralized tissue volume (CV) instead of bone volume (BV).

### Static parameters

Only one static parameter, mineralized tissue fraction (CV/TV) was analyzed for this study as all other typical bone parameters would be misleading when looking at mixture of bone and a mineralizing biomaterial. The results from this study were compared to the BV/TV that was achieved with pure Zoledronate-loaded hydrogel and in control animals without biomaterial in the earlier study. This comparison allows an evaluation of the nHA influence. The CV/TV values were normalized to the first microCT scan (day 3) to rule out a bias caused by the nHA that was visible on microCT scans already at day 3. The analysis of the CV/TV in the layer next to the screw surface (0-368 µm) showed no difference between the nHA-Zol-Gel-group and the group that released the drug from pure hydrogel (Zol-Gel-group) (Fig. 6). In the second layer (368-736 µm) the CV/TV gain was around 50% higher in the nHA-Zol-Gel-group than in the Zol-Gel-group starting from day 17. An important standard deviation however prevented a statistical significance of the results. This difference diminished to around 30% in the third layer (736-1104 µm), a significant difference again could not be shown due to a high variability of the results. No difference at all was visible in the fourth layer (1104-1472 µm). For the comparison with the nHA-Gel-group, the control group of the earlier study has been chosen as baseline because pure hyaluronic acid hydrogel had been shown to not at all influence the peri-implant bone healing/remodeling compared to control. Close to the screw (0-368 µm), the nHA in the hydrogel caused a mineralized tissue fraction gain that was around 30% higher than in the control group during the full experimental period. This difference was however not statistically significant. In the second layer (368-736 µm), the CV/TV doubled between day 3 and day 10 what was comparable to the Zoledronate-treated groups and significantly higher than in the control group which showed a constant CV/TV. Later on the difference between the two groups diminished and was only marginal at the end of the experimental period. A similar development could be observed in the third layer, where the initial gain in CV/TV was only 50% and the difference between nHA-Gel-group and control equaled out by day 17. No difference between nHA-Gel-group and the control group could be found in the outermost layer (1104-1472 µm).

### Dynamic parameters

The analysis of the dynamic parameters gives information about bone formation and resorption processes. In the case of the present study, it is not clear which part of the appearing mineralized structure was new bone and which part was mineralizing biomaterial, therefore we measured a general mineralization rate instead of a bone formation rate. The same applied for the bone resorption rate which was named demineralization rate for this study and included bone resorption and biomaterial degradation. A 3D rendering of one example from each study group (Fig. 7) gave a good overview over the mineralization and demineralization processes. In both groups we found a significant mineralization around the screw between day 3 and day 10, which was followed by a pronounced demineralization in the nHA-Gel-group. Also the bone around the implantation site was gradually resorbed. The nHA-Zol-Gel-group, on the contrary, showed only very little demineralization in the analysed region. Those findings were also represented in the measured mineralization and demineralization rate (Fig. 8 and 9). No differences in the mineralization rate could be found between the two groups from this study and the two groups without nHA from the former experiment in direct contact with the screw (layer 0-368 µm). In the second layer (368-736 µm), however, between day 3 and day 10 there was a highly significant difference between the nHA-Gel-group and the control group and a small trend to a higher mineralization rate in the nHA-Zol-Gel-group compared to the group that released the drug directly from the hydrogel (Zol-Gel-group). Those differences equaled out by day 10. The same situation could be found also in the outer two layers (736-1472 µm). When looking at the bone resorption rate, we saw the typical difference between the Zoledronate and the non-Zoledronate groups. The bone resorption/demineralization peak between days 10 and 31 that is characteristic for the drug-free groups was completely inhibited by the locally delivered Zoledronate. The demineralization rate of the nHA-Zol-Gel-group did not differ at any time and in any region from the one of the Zol-Gel-group. When comparing the nHA-Gel-group with the control group, there was an unexpected trend to a higher demineralization rate in the nHA-Group in the outer two layers (736-1472 µm).

### Example 4

### Histology

The histological results confirmed the earlier finding that the used hyaluronic acid hydrogel is fully degraded after 58 days of implantation without leaving any visible residues (Fig. 10). All screws showed a good osteointegration without any inflammation reaction or fibrotic tissue encapsulation. Surprisingly, in both nHA-Gel- and nHA-Zol-Gel group there were regions of mineralizations present within the bone. Those granule shaped mineralizations had a diameter of up to 50 µm in the nHA-Gel-group and extended across wide areas in the nHA-Gel group. The X-rays taken of the histology slides indicate that those mineralizations can be denser than bone. It can be excluded that those regions represent remaining nHA particles as those particles have a diameter of less than 200 nm and the observed mineralizations are much bigger. In the nHA-Gel group, only small amounts of those mineralizations were found in islands of new bone located mainly close to the screw tip, the region where the hydrogel cumulates during screw insertion. The mineralizations were completely embedded in the bone matrix and the resulting constructs showed a trabecular structure. Large amounts of multinucleated cells such as macrophages and giant foreign body cells were present in the bone marrow around these bone-mineralization islands indicating an on-going resorption of the biomaterial. No new bone formation could be detected in this group beside a thin layer close to the screw surface and the bone close to the biomaterial. Almost no trabecular bone was left around the screw in the nHA-Gel animals 13 weeks after OVX. The nHA-Zol-Gel-group showed a completely different situation. Significantly larger mineralizations could be found mainly close to the screw tip but also between the threads. The remaining biomaterial had a porous structure and was partly penetrated by bone tissue. In contrast to what was seen in the nHA-Gel-group, the bone-mineralization constructs was rather compact and did not show a trabeculae-like structure. Smaller amounts of macrophages but no giant foreign body cells could be detected in the bone marrow close to the biomaterial what confirms the inhibiting effect of Zoledronate. In general, a lot more trabecular bone could be found close and far from the screw in the nHA-Zol-Gel-group compared to the nHA-Gel group. New bone formed close to and far from the screw surface and especially also at the interface to the biomaterial. A significant periosteal callus formation was also seen in the nHA-Zol-Gel-group that was not present to this extent in the nHA-Gel-group.

The much unexpected finding of this study was the rapid in vivo mineralization of the hydrogel that happened independently from the bisphosphonate in both groups. It is known that calcium phosphate particles can act as nucleation sites in hydrogel matrices that promote a HA precipitation. This phenomenon has been investigated in the past mainly in vitro with simulated body fluids but the present invention showed for the first time such a rapid mineralization in vivo. The mineralizations that were found in the present study showed mainly a granule-like structure with a particle size of up to 50 µm formed by hydroxyapatite deposition starting from the nHA particles as nucleation points. In the nHA-Zol-Gel-group, the granules seemed to have fused even to larger mineralized regions as shown with histology. A complete integration of the newly formed granules into the bone matrix without any signs of inflammation or foreign body reaction confirmed their excellent biocompatibility and osteoconductivity. When comparing the nHA-Gel group of this study with a control group of a former study that received only screw (data not shown), a significant difference in the early mineralization in the three outer analyzed layers (736-1472µm) was observed. The difference diminished over time and stayed only as a trend in the inner two layers. The explanation for this phenomenon is an initial rapid mineralization of the biomaterial followed by its simultaneously occurring degradation and penetration by bone tissue as shown by structural changes on the time-lapsed microCT scans and the terminal histology results. Large amounts of macrophages and giant foreign body cells that could be found in the nHA-Gel-group shows an efficient biodegradation of the hydroxyapatite. The obvious mineralizations of the biomaterial complicated the evaluation of the drug effect on peri-implant bone. The in vivo microCT imaging does not allow a differentiation between newly formed woven bone and mineralized hydrogel. However, there is a strong trend to more tissue mineralization in the two central layers of the analyzed region (368-736 µm) that appears early and remains during the whole study time. Contributors to this gain of mineralized tissue could be on one side the new woven bone formation and on the other side the biomaterial mineralization. In inventors' earlier studies it was shown that the discovered boost in early peri-implant bone formation was caused by an initial "flush" of all bone cells with bisphosphonate. With the here presented release study, it can be seen that this flush does not occur when nHA is included in the hydrogel matrix as the Zoledronate is absorbed to the particles. It can be presumed that the release of the comparatively huge nHA particles is significantly slower than the release of the small drug molecules. As the non-phagocytic osteoblasts are unlikely to incorporate Zoledronate loaded-particles, less influence on bone formation can be assumed for the nHA-Zol-Gel-group compared to the situation when Zoledronate is delivered from pure hydrogel. Nevertheless, little, but still significant differences could be also shown for the mineralization rate between the nHA-Gel-group and the nHA-Zol-Gel-group. From day 7 to 10 there was a higher mineralization rate seen in the nHA-Gel-group and from day 31-58 in the nHA-Zol-Gel-group. As a conclusion the combination Zoledronate-nHA must influence bone formation and/or biomaterial mineralization in a different way than pure nHA. When looking at the demineralization rate, the strong inhibitory effect of Zoledronate becomes obvious in the highly significant differences between the nHA-Gel-group and the nHA-Zol-Gel-group. The microCT study and histology revealed that the drug did not only inhibit bone resorption but also biomaterial degradation as can be seen by the absence of macrophages and giant foreign body cells and much larger amounts of mineralizations remaining in the nHA-Zol-Gel-group. Histology also shows that the bone/biomaterial-construct in the nHA-Gel-group had already been remodeled to a trabecular structure whereas it is still rather compact in the nHA-Zol-Gel-group. Surprisingly, there is no difference in demineralization rate between the Zol-Gel-group from the former study and the nHA-Zol-Gel-group from the present study even very distant from the screw (1104-1472 µm) where the bone is very unlikely to be in contact with the hydrogel. Therefore there must be either a release of the drug-loaded particles or a release of the drug alone as it can be caused by the resorptive activity of osteoclasts or macrophages.

The tested biomaterials are easy to apply to bone due to their soft consistency what makes them perfect for an application to irregular shaped cavities like an implant bed or difficult to access bone defects. These materials have also been shown to calcify rapidly in vivo and to form a kind of osteoconductive mineral scaffold. This biodegradable scaffold is later on penetrated by bone cells and included in newly forming bone. The hydrogel with nHA and Zoledronate could be favorable for the repair of bone defects that require at the same time a preservation of the surrounding bone matrix.

## Claims

1. An in vivo mineralizing composition comprising a plurality of calcium phosphate solid particles loaded with an active agent and dispersed in a hydrogel carrier comprising hyaluronic acid, **characterized in that** the particles have a mean size comprised between 150nm and 500nm.

2. The composition of claim 1, wherein the solid particles are selected from the group consisting of crystals, granules, round particles, spherical particles or any combination thereof.

3. The composition of claim 1, wherein the active agent is an anti-resorptive agent.

4. The composition of claims 1 to 3, wherein the active agent consists of or includes a bisphosphonate.

5. The composition of claim 4, wherein the bisphosphonate includes or consists of Zoledronate.

6. The composition of claims 1 to 5, wherein the calcium phosphate solid particles are hydroxyapatite particles.

7. The composition of claims 1 to 6, **characterized in that** it is in injectable hydrogel, form.

8. A pharmaceutical formulation comprising the composition of claims 1 to 7.

## Patentansprüche

1. In-vivo-Mineralisierungszusammensetzung, umfassend mehrere mit einem Wirkstoff beladene und in einem Hyaluronsäure umfassenden Hydrogel-Träger dispergierte feste Calciumphosphatpartikel, **dadurch gekennzeichnet, dass** die Partikel eine mittlere Größe zwischen 150 nm und 500 nm aufweisen.

2. Zusammensetzung nach Anspruch 1, wobei die festen Partikel aus der aus Kristallen, Granulat, runden Partikeln, sphärischen Partikeln oder einer beliebigen Kombination davon bestehenden Gruppe ausgewählt sind.

3. Zusammensetzung nach Anspruch 1, wobei es sich bei dem Wirkstoff um ein antiresorptives Mittel handelt.

4. Zusammensetzung nach einem der Ansprüche 1 bis 3, wobei der Wirkstoff aus einem Bisphosphonat besteht oder ein Bisphosphonat einschließt.

5. Zusammensetzung nach Anspruch 4, wobei das Bisphosphonat Zoledronat einschließt oder daraus besteht.

6. Zusammensetzung nach einem der Ansprüche 1 bis 5, wobei es sich bei den festen Calciumphosphatpartikeln um Hydroxyapatitpartikel handelt.

7. Zusammensetzung nach einem der Ansprüche 1 bis 6, **dadurch gekennzeichnet, dass** sie in Form eines injizierbaren Hydrogels vorliegt.

8. Pharmazeutische Formulierung, umfassend die Zusammensetzung nach einem der Ansprüche 1 bis 7.

## Revendications

1. Composition de minéralisation *in vivo* comprenant une pluralité de particules solides de phosphate de calcium chargées avec un agent actif et dispersées dans un support d'hydrogel comprenant de l'acide hyaluronique, **caractérisée en ce que** les particules possèdent une taille moyenne comprise entre 150 nm et 500 nm.

2. Composition selon la revendication 1, les particules solides étant choisies dans le groupe constitué par des cristaux, des granules, des particules rondes, des particules sphériques et une quelconque combinaison correspondante.

3. Composition selon la revendication 1, l'agent actif étant un agent anti-résorption.

4. Composition selon les revendications 1 à 3, l'agent actif étant constitué d'un, ou comportant un, bisphosphonate.

5. Composition selon la revendication 4, le bisphosphonate comportant, ou étant constitué de Zolédronate.

6. Composition selon les revendications 1 à 5, les particules solides de phosphate de calcium étant des particules d'hydroxyapatite.

7. Composition selon les revendications 1 à 6, **caractérisée en ce qu'**elle est sous forme d'hydrogel injectable.

8. Formulation pharmaceutique comprenant la composition selon les revendications 1 à 7.
